# EUROPEAN PATENT SPECIFICATION

(11) **EP 1 277 486 B1**
(45) Date of publication and mention of the grant of the patent: **13.10.2010**
(21) Application number: 02011587.9
(22) Date of filing: 27.05.2002
(51) Int. Cl.: A61M 5/14

(54) **Disposable preassembled device particularly for photodynamic therapy**
Vorkonfektionierte Wegwerfvorrichtung insbesondere für die photodynamische Therapie
Dispositif jetable préassemblé en particulier pour la thérapie photodynamique

(30) Priority: 05.06.2001 IT MO20010114
(43) Date of publication of application: 22.01.2003
(73) Proprietor: ARIES S.r.l., 41037 Mirandola (Modena) (IT)
(72) Inventor: Mantovani, Mauro, 41037 Mirandola (Modena) (IT)
(74) Representative: Modiano, Micaela Nadia

(56) References cited:
- EP-A- 1 161 960
- US-A- 2 893 395
- US-A- 4 116 646
- US-A- 4 573 974
- US-A- 4 585 435
- US-A- 4 941 875
- US-A- 5 183 472
- US-A- 5 840 058

## Description

The present invention relates to a disposable preassembled device, for photodynamic therapy.

Photodynamic therapy has long been used to treat eye disorders, mainly consisting of dry or exudative maculopathies.

Substantially, since these disorders involve the onset of visual dark spots that reduce the viewing field of the eyes which are caused by the formation of capillaries under the retina (the so-called neovascular membrane), the therapy that is currently preferably used to reduce or eliminate the harmful effects of these disorders is the injection of a photosensitive-activation drug in the venous circuit of the patient, at a concentration that allows it to reach such capillaries to be eliminated, followed by activation of said drug with a source of halogen light, such as the light emitted by a laser device; the drug, by reacting, dries such capillaries, reducing their overall volume considerably and improving the viewing field of the eye.

In practice, in this manner, and by repeating the therapeutic application for a few sessions at intervals of a few days, it is possible to at least reduce considerably the effect of these disorders if not eliminate it completely.

The drug that is used (verteporfin) has the drawback of being extremely expensive; accordingly, it is necessary to recover the volumes thereof retained inside the devices used for administration.

This recovery entails a number of problems, a main one being the preservation of the sterility of the system, i.e., preventing the drug from making contact with the outside environment and becoming loaded with bacteria.

A second problem is the risk of accidental loss of the drug when it is recovered with empirical methods and makeshift or improvised equipment.

Furthermore, in order to be able to administer the necessary doses to patients, multiple packages of drug have been used, up to now, because part of the drug, during administration, is retained within the tubing that composes the devices used.

In fact the manufacturers of said drug package it in calibrated doses, which, due to the significant length of said tubing, are reduced by said retention; in order to complete administration in a correct therapeutic quantity, the paramedical personnel must therefore open a new package to be used as a propulsion means.

US 4,116,646 discloses a piggyback arrangement for administering two solutions to a patient, comprising a Y connection having an outlet connected to a final means for administration to a patient and two inlets connected to two tubings from two respective hung containers of the solutions, respective filter units being interposed between each container and the Y connection.

The aim of the present invention is to solve the above described problem of the known art by providing a disposable preassembled device for photodynamic therapy, that allows to perform the therapy without wasting the drug (verteporfin).

This aim and other objects that will become better apparent hereinafter are achieved by the disposable preassembled device for photodynamic therapy according to claims 1 and 2.

Further characteristics and advantages of the present invention will become better apparent from the detailed description of a preferred but not exclusive embodiment of a disposable preassembled device for photodynamic therapy, illustrated only by way of non-limitative example in the accompanying drawings, wherein:

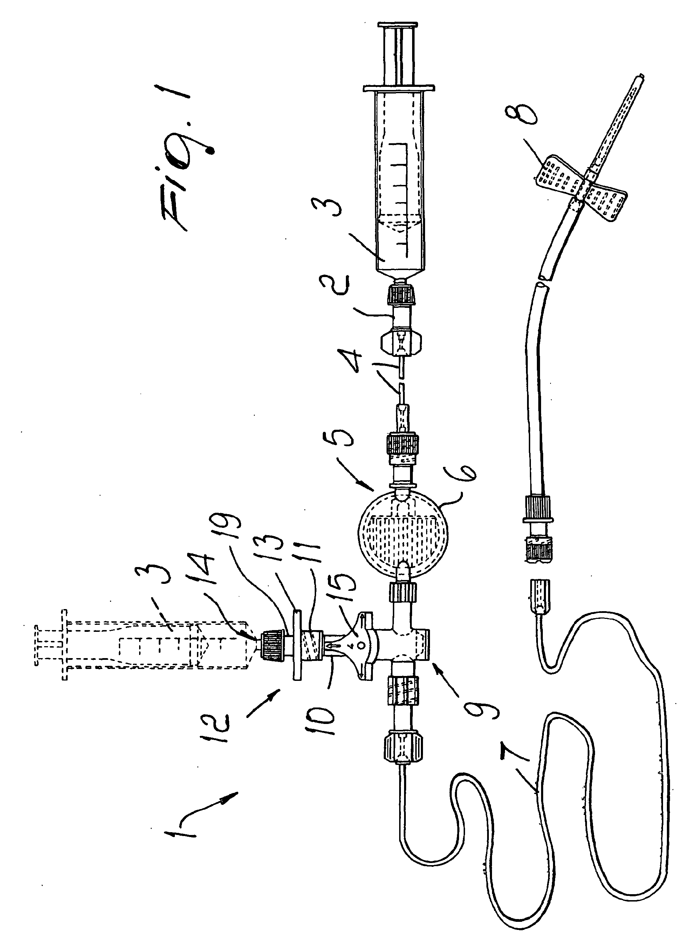

Figure 1 is a view of the disposable preassembled device for photodynamic therapy, according to the invention;

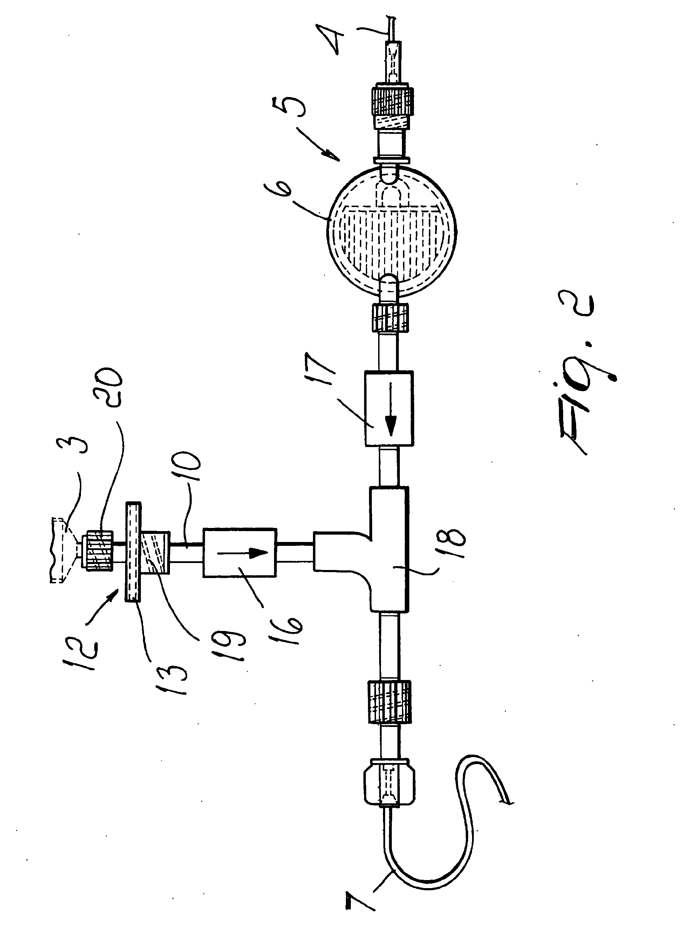

Figure 2 is a view of the disposable preassembled device for photodynamic therapy, according to the invention, in an alternative embodiment.

With reference to the figures, the reference numeral 1 generally designates a disposable preassembled device for photodynamic therapy, which comprises an inlet 2 to which a drug dispensing syringe 3 can be connected.

A first portion of tubing 4 leads out from the inlet 2, which is preferably provided with a Luer-type connector, and leads into a first filtering means 5, which in practice is a flat filter 6 with a porosity of 1.2 microns; a second portion 7 of tubing is connected to the output of said filtering means for connection to a final means, for example a butterfly needle 8, for administration to a patient.

Directly downstream of the flat filter 6 a shunt means 9 is provided for connection to a portion of shunted tubing 10 for connection to the outlet 11 of a second filtering means 12, usually again constituted by a flat filter 13, which has a porosity between 0.2 and 17 microns and has, at its inlet, a protected connector 14.

The shunt means 9 is constituted by a three-way cock 15, which is adapted to alternately connect said first or second filtering means 5 or 12 to the second portion of tubing 7.

The shunt means 9 can also be constituted, in an alternative embodiment of the invention, by two flow control valves, designated by the reference numerals 16 and 17 respectively, both of the one-way type, which are arranged respectively on the first portion of tubing 4 and on the shunted tubing portion 10; said valves are advantageously connected one another by way of a Y-shaped union 18 and allow flow toward the second portion of tubing 7.

The protected connector 14 is preferably constituted by a so-called injection point 19, provided with a closure cap 20.

The operation of the invention is as follows: the operator loads the syringe 3 with the predefined dose of drug to be used on the patient, then connects its outlet, without the needle, to the inlet 2.

The operator then inserts the needle 8 in the vein of the patient and, by acting on the syringe 3, starts to dispense the pre-calibrated dose of drug.

Such dose passes through the flat filter 6, is purified of any microimpurities, and reaches the cock 15, which is set beforehand so as to connect the first portion of tubing 4 to the second portion of tubing 7, which is usually much longer than the first one to allow a certain freedom of motion to the patient during administration.

At the same time, the shunted tubing 10 is blocked by said cock 15, which thus prevents any rise of the drug back toward the shunted line 10.

If the cock 15 is replaced by the one-way valves 16 and 17, the operation of the invention is similar.

The drug injected through the inlet 2 in fact passes from the first portion of tubing 4 to the filter 6 and then to the second portion 7 without being able to rise back, except for a few millimeters, toward the shunted tubing 10, being prevented from doing so by the valve 16, which allows flow only in the opposite direction.

The injected drug then reaches the patient, and when the dose of drug is completely depleted in the syringe 3, said syringe is disconnected from the inlet 2.

A significant residual volume of drug, which is part of the preset dose and is not injected into the patient, remains nonetheless inside the second portion of tubing 7.

In order to complete administration, the operator fills the syringe 3 with an appropriate solution and, after removing the closure cap 20, inserts the male cone of the syringe 3 into the female cone of the injection point 19 of the flat filter 13; he then turns the cock 15 to connect one another the portion of shunted tubing 10 and the second portion 7, thus blocking the first portion 4.

He then injects the solution until said solution, by passing in the second portion 7, after passing through the flat filter 13, fully pushes the residual volume of drug that remains in said second portion of tubing 7 toward the patient.

In the alternative embodiment, operation is similar, the only difference being that the operator does not even have to change the position of anything (cocks 15 or the like), since the one-way valves 16 and 17 themselves allow the forced flow of the physiological solution from the shunted portion of tubing 10 toward the second portion 7.

In this manner, administration of the preset dose of drug occurs completely and aseptically, without wasting any more drug.

In practice it has been found that the described invention achieves the intended aim and objects.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A disposable preassembled device for administering a complete dose of drug for photodynamic therapy, of the type comprising an inlet (2) for connection to a drug dispensing syringe (3), a first portion of tubing (4) leading out therefrom and into a first filtering means (5), said disposable preassembled device comprising:
a second filtering means (12) provided at its inlet with a protected connector (14);
a portion of shunted tubing (10) connected to the outlet (11) of said second filtering means (12);
a shunt means (9) provided downstream of said first filtering means (5) and having a first inlet connected to the outlet of said first filtering means (5),
a second inlet connected to said portion of shunted tubing (10), and
an outlet connected to a second portion of tubing (7) which is connectable to a final means (8) for administration to a patient,
said shunt means (9) being constituted by a three-way cock (15) for allowing flow toward said second portion of tubing (7) alternatively from said first and second filtering means, through the alternative connection of said first (5) or second (12) filtering means to said second portion of tubing (7), so that residual drug from the first filtering means (5) can be removed from said second portion of tubing (7) through flow from the second filtering means (12) and the portion of shunted tubing (10) toward the final administering means (8).

2. A disposable preassembled device for administering a complete dose of drug for photodynamic therapy, of the type comprising an inlet (2) for connection to a drug dispensing syringe (3), a first portion of tubing (4) leading out therefrom and into a first filtering means (5), said disposable preassembled device comprising:
a second filtering means (12) provided at its inlet with a protected connector (14);
a portion of shunted tubing (10) connected to the outlet (11) of said second filtering means (12);
a shunt means (9) provided downstream of said first filtering means (5) and having a first inlet connected to the outlet of said first filtering means (5),
a second inlet connected to said portion of shunted tubing (10), and
an outlet connected to a second portion of tubing (7) which is connectable to a final means (8) for administration to a patient,
said shunt means (9) being constituted by two flow control valves (16, 17), both of which are of the one-way type and are arranged respectively on said first portion of tubing (4) and on said portion of shunted tubing (10), said flow control valves (16, 17) being connected one another by way of a Y-shaped union (18) and both allowing flow toward said second portion of tubing (7) for connection to a final administration means, so that residual drug from the first filtering means (5) can be removed from said second portion of tubing (7) through flow from the second filtering means (12) and the portion of shunted tubing (10) toward the final administering means (8).

3. The device according to claim 1, **characterized in that** said protected connector (14) is constituted by a so-called injection point (19) provided with a closure cap (20).

4. The device according to claims 1 and 2, **characterized in that** said first filtering means (5) has an average porosity between 1.1 and 1.3 microns and **in that** said second filtering means (12) has an average porosity between 0.2 and 17 microns.

## Patentansprüche

1. Eine vorkonfektionierte Einwegvorrichtung zur Verabreichung einer vollständigen Dosis eines Arzneimittels für die photodynamische Therapie, von der Art, die Folgendes umfasst:
einen Einlass (2) zur Verbindung mit einer Arzneimittel abgebenden Spritze (3), einen ersten Röhrenleitung-Abschnitt (4), der von dort hinaus und in ein erstes Filtermittel (5) führt,
wobei die vorkonfektionierte Einwegvorrichtung Folgendes umfasst:
ein zweites Filtermittel (12), das an seinem Einlass mit einem geschützten Verbindungsstück (14) versehen ist,
einen Ableitungs-Röhrenleitung-Abschnitt (10), der mit dem Auslass (11) des zweiten Filtermittels (12) verbunden ist,
ein Ableitungsmittel (9), das stromabwärts von dem ersten Filtermittel (5) bereitgestellt ist und das einen ersten Einlass hat, der mit dem Auslass des ersten Filtermittels (5) verbunden ist,
einen zweiten Einlass, der mit dem Ableitungs-Röhrenleitung-Abschnitt (10) verbunden ist, und
einen Auslass, der mit einem zweiten Röhrenleitung-Abschnitt (7) verbunden ist, der mit einem End-Mittel (8) für die Verabreichung an einen Patienten verbunden werden kann,
wobei das Ableitungsmittel (9) aus einem Dreiweghahn (15) besteht, um den Fluss zu dem zweiten Röhrenleitung-Abschnitt (7) alternativ von dem ersten und dem zweiten Filtermittel zu ermöglichen, durch die alternative Verbindung des ersten (5) oder des zweiten (12) Filtermittels mit dem zweiten Röhrenleitung-Abschnitt (7), so dass restliches Arzneimittel aus dem ersten Filtermittel (5) aus dem zweiten Röhrenleitung-Abschnitt (7) durch Strömung aus dem zweiten Filtermittel (12) und dem Abzweigungs-Röhrenleitung-ABschnitt (10) zum End-Verabreichungsmittel (8) hin entfernt werden kann.

2. Eine vorkonfektionierte Einwegvorrichtung zur Verabreichung einer vollständigen Dosis eines Arzneimittels für die photodynamische Therapie, von der Art, die Folgendes umfasst:
einen Einlass (2) zur Verbindung mit einer Arzneimittel abgebenden Spritze (3), einen ersten Röhrenleitung-Abschnitt (4), der von dort hinaus und in ein erstes Filtermittel (5) führt,
wobei die vorkonfektionierte Einwegvorrichtung Folgendes umfasst:
ein zweites Filtermittel (12), das an seinem Einlass mit einem geschützten Verbindungsstück (14) versehen ist,
einen Ableitungs-Röhrenleitung-Abschnitt (10), der mit dem Auslass (11) des zweiten Filtermittels (12) verbunden ist,
ein Ableitungsmittel (9), das stromabwärts von dem ersten Filtermittel (5) bereitgestellt ist, das einen ersten Einlass hat, der mit dem Auslass des ersten Filtermittels (5) verbunden ist,
einen zweiten Einlass, der mit dem Ableitungs-Röhrenleitung-Abschnitt (10) verbunden ist, und
einen Auslass, der mit einem zweiten Röhrenleitung-Abschnitt (7) verbunden ist, der mit einem End-Mittel (8) für die Verabreichung an einen Patienten verbunden werden kann,
wobei das Ableitungsmittel (9) aus zwei Durchflussregelventilen (16, 17) besteht, die beide vom Einwegtyp und an dem ersten Röhrenleitung-Abschnitt (4) beziehungsweise an dem Ableitungs-Röhrenleitung-Abschnitt (10) angeordnet sind, wobei die Durchflussregelventil (16, 17) miteinander durch einen Y-förmigen Anschluss (18) verbunden sind und beide die Strömung zu dem zweiten Röhrenleitung-Abschnitt (7) zur Verbindung mit einem End-Verabreichungsmittel ermöglichen, so dass restliches Arzneimittel aus dem ersten Filtermittel (5) aus dem zweiten Röhrenleitung-Abschnitt (7) durch Strömung aus dem zweiten Filtermittel (12) und dem Ableitungs-Röhrenleitung-Abschnitt (10) zum End-Verabreichungsmittel (8) hin entfernt werden kann.

3. Die Vorrichtung gemäß Anspruch 1, **dadurch gekennzeichnet, dass** das geschützte Verbindungsstück (14) aus einem so genannten Injektionspunkt (19), der mit einer Verschlusskappe (20) versehen ist, besteht.

4. Die Vorrichtung gemäß Anspruch 1 und 2, **dadurch gekennzeichnet, dass** das erste Filtermittel (5) eine durchschnittliche Porosität zwischen 1,1 und 1,3 Mikron hat, und dadurch, dass das zweite Filtermittel (12) eine durchschnittliche Porosität zwischen 0,2 und 17 Mikron hat.

## Revendications

1. Dispositif pré-assemblé jetable pour administrer une dose complète de médicament pour une thérapie photodynamique, du type comprenant une entrée (2) pour le raccordement à une seringue de délivrance de médicament (3), une première portion de tubulure (4) en sortant et conduisant dans un premier moyen de filtration (5), ledit dispositif pré-assemblé jetable comprenant :
un deuxième moyen de filtration (12) disposé au niveau de son entrée avec un raccord protégé (14) ;
une portion de tubulure dérivée (10) raccordée à la sortie (11) dudit deuxième moyen de filtration (12) ;
un moyen de dérivation (9) disposé en aval dudit premier moyen de filtration (5) et ayant une première entrée raccordée à la sortie dudit premier moyen de filtration (5),
une deuxième entrée raccordée à ladite portion de tubulure dérivée (10), et
une sortie raccordée à une deuxième portion de tubulure (7) qui peut être raccordée à un moyen final (8) pour l'administration à un patient,
ledit moyen de dérivation (9) étant constitué d'un robinet à trois voies (15) pour permettre l'écoulement vers ladite deuxième portion de tubulure (7) alternativement à partir dudit premier moyen de filtration et dudit deuxième moyen de filtration, à travers le raccordement alternatif dudit premier (5) ou deuxième (12) moyen de filtration vers ladite deuxième portion de tubulure (7), de sorte que le médicament résiduel en provenance du premier moyen de filtration (5) peut être enlevé de ladite deuxième portion de tubulure (7) par l'écoulement en provenance du deuxième moyen de filtration (12) et de la portion de tubulure dérivée (10) vers le moyen d'administration final (8).

2. Dispositif pré-assemblé jetable pour administrer une dose complète de médicament pour une thérapie photodynamique, du type comprenant une entrée (2) pour le raccordement à une seringue de délivrance de médicament (3), une première portion de tubulure (4) en sortant et conduisant dans un premier moyen de filtration (5), ledit Dispositif pré-assemblé jetable comprenant :
un deuxième moyen de filtration (12) disposé au niveau de son entrée avec un raccord protégé (14) ;
une portion de tubulure dérivée (10) raccordée à la sortie (11) dudit deuxième moyen de filtration (12) ;
un moyen de dérivation (9) disposé en aval dudit premier moyen de filtration (5) et ayant une première entrée raccordée à la sortie dudit premier moyen de filtration (5),
une deuxième entrée raccordée à ladite portion de tubulure dérivée (10), et
une sortie raccordée à une deuxième portion de tubulure (7) qui peut être raccordée à un moyen final (8) pour l'administration à un patient,
ledit moyen de dérivation (9) étant constitué de deux vannes de commande d'écoulement (16, 17), dont les deux sont du type à retenue et étant agencées respectivement sur ladite première portion de tubulure (4) et sur ladite portion de tubulure dérivée (10), lesdites vannes de commande d'écoulement (16, 17) étant raccordées l'une à l'autre au moyen d'un raccord en Y (18), et toutes deux permettant un écoulement vers ladite deuxième portion de tubulure (7) pour le raccordement à un moyen d'administration final de sorte que le médicament résiduel en provenance du premier moyen de filtration (5) peut être enlevé de ladite deuxième portion de tubulure (7) par un écoulement en provenance du deuxième moyen de filtration (12) et de la portion de tubulure dérivée (10) en direction du moyen d'administration final (8).

3. Dispositif selon la revendication 1, **caractérisé en ce que** ledit raccord protégé (14) est constitué de ce qu'on appelle un point d'injection (19) muni d'un capuchon de fermeture (20).

4. Dispositif selon les revendications 1 et 2, **caractérisé en ce que** ledit premier moyen de filtration (5) a une porosité moyenne entre 1,1 et 1,3 micron, et **en ce que** ledit deuxième moyen de filtration (12) a une porosité moyenne entre 0,2 et 17 microns.
